# EUROPÄISCHE PATENTSCHRIFT

(11) **EP 2 424 445 B1**
(45) Veröffentlichungstag und Bekanntmachung des Hinweises auf die Patenterteilung: **09.01.2019**
(21) Anmeldenummer: 09753039.8
(22) Anmeldetag: 11.11.2009
(51) Int. Cl.: A61B 17/08, A61B 17/04

(54) **WUNDVERSCHLUSS**
WOUND CLOSURE DEVICE
SYSTÈME DE FERMETURE DE PLAIE

(30) Priorität: 29.04.2009 DE 102009020763
(43) Veröffentlichungstag der Anmeldung: 07.03.2012
(73) Patentinhaber: Aesculap AG, 78532 Tuttlingen (DE)
(72) Erfinder: BARGON, Rainer, 88512 Mengen (DE); ODERMATT, Erich, 8200 Schaffhausen (CH); STÖVER, Bernd, 22829 Schenefeld (DE)
(74) Vertreter: Patentanwälte Ruff, Wilhelm, Beier, Dauster & Partner mbB
(86) Internationale Anmeldenummer: PCT/EP2009/008029
(87) Internationale Veröffentlichungsnummer: WO 2010/124712

(56) Entgegenhaltungen:
- EP-A1- 1 462 075
- WO-A1-2008/143689
- WO-A2-02/38096
- DE-U1-202006 015 861
- US-A- 4 825 866
- US-A- 5 009 663
- US-A1- 2004 193 216
- US-B1- 6 176 868

## Beschreibung

Die Erfindung betrifft einen verstellbaren Wundverschluss zum Verschließen von Wunden.

Als Wunde wird in der Regel die Trennung von Gewebe an äußeren oder inneren Körperoberflächen bezeichnet. Solche Wunden bei Mensch oder Tier können thermisch (Verbrennungen, Erfrierungen) oder chemisch (z.B. Verätzungen) verursacht sein. Häufig handelt es sich jedoch um mechanisch verursachte Wunden, die jedoch nicht nur zufällig verursacht sind, sondern beispielsweise bei Operationen zwingend in Kauf genommen werden müssen. Im letzteren Fall spricht man dann auch von Inzisionen, d.h. von bewusst vorgenommenen Einschnitten in den menschlichen Körper, beispielsweise im Zuge von chirurgischen Operationen.

In vielen Fällen müssen die Wunden, insbesondere nach Operationen, zumindest vorübergehend durch irgendwelche Hilfsmittel verschlossen oder gegen die Umgebung abgeschlossen werden. Dies dient in erster Linie dazu, den Eintritt von körperfremden Materialien, insbesondere von Erregern zu verhindern und die Wundheilung zu beschleunigen.

Entsprechende Wundverschlüsse sind dem Fachmann selbstverständlich bereits in einer Vielzahl von Ausführungen bekannt. Sie reichen von Pflastern über Verbände bis hin zu speziellen Wundverschlüssen für bestimmte Anwendungen. Zu nennen ist natürlich auch das Nähen, bei dem die Wunde mit Hilfe einer Nadel sowie resorbierbaren oder nicht resorbierbaren Fäden verschlossen wird.

Die genannten und andere Wundverschlüsse sind dabei in der Regel entweder für den Hautverschluss oder für den Wundverschluss innerhalb des Körpers, z.B. den sogenannten Faszienverschluss, vorgesehen.

Im Zusammenhang mit dieser Erfindung soll im Vorfeld nicht im Detail auf die bisher bekannten Lösungen zum Wundverschluss eingegangen werden. So sind natürlich Wundverschlüsse auf Basis von Klammern, von Nahtmaterial oder von Klebern seit vielen Jahrzehnten bekannt. Auch die Anwendung von Klebestreifen/Pflasterstreifen ist mit einer Vielzahl von Ausführungen bereits dokumentiert. Sogar vergleichsweise exotische Wundverschlüsse wie Schraubverschlüsse oder Magnetverschlüsse wurden diskutiert und teilweise auch realisiert.

Bereits beschrieben sind auch nahtlose Wundverschlüsse, bei denen am Wundrand Verankerungselemente fixiert und dann über eine Verschnürung gegeneinander festgezogen werden. Dadurch werden die Wundränder aufeinander zubewegt und so die Wunde verschlossen. Offenbart ist eine solche Ausführung in der US-B-7,429,265.
Nachteilig an dieser Ausführung ist, dass eine Vielzahl von Verankerungselementen (Halteelementen) am Wundrand angebracht werden muss, um die Wunde über ihre gesamte Länge mit einer im wesentlichen konstanten Spannung zu verschließen. Außerdem werden in der genannten US-Schrift die Halteelemente mit Hilfe von Dornenfortsätzen im Gewebe verankert, was zu einer zusätzlichen Verletzung des Gewebes führt. Schließlich bedingt die Konstruktion dieser Ausführung, dass das Anlegen des Wundverschlusses an der Wunde zeitaufwändig ist.

Weiter sind auch bereits Wundverschlüsse bekannt, die auf Klettverschlüssen basieren oder Klettverschlüsse einsetzen. In diesem Zusammenhang soll auf die US-A-5,876,365, die US-B-7,414,168 und die US-A-4,825,866 verwiesen werden.

Die US-A-5,876,365 beschreibt einen Klebeverband, bei dem eine vom Verband rundum begrenzte Öffnung vorgesehen ist, durch welche die Wunde von oben her zugänglich bleibt. Zum reversiblen Verschließen der Öffnung ist ein flächiges, geschlossenes Abdeckelement vorgesehen, das am Rand der Öffnung mit Hilfe von Klettstreifen befestigbar ist.

Die US-B-7,414,168 beschreibt ein zweiteiliges Klettverschlusssystem zur Anwendung bei Lazerationen (Einrissen) oder Inzisionen, das aus zwei Pflastern mit einer Klebeunterseite besteht. Die beiden Pflaster werden auf den gegenüberliegenden Seiten einer Wunde angebracht. Sie weisen seitlich Streifen auf, die über die Wunde hinweg mit Hilfe eines Klettverschlusses auf der Oberfläche des gegenüberliegenden Pflasters befestigt werden können. Dadurch werden die Wundränder aufeinander zugezogen und die Wunde wird verschlossen.

In der US-A-4,825,866 sind ebenfalls auf gegenüberliegenden Seiten der Wunde Klebestreifen nach Art von Pflastern angeordnet, die hier allerdings mit separaten parallel angeordneten Streifenelementen gegeneinander gezogen und fixiert werden. Weiter sind an den Klebestreifen lateral angeordnete verformbare Röhrchen vorgesehen, mit deren Hilfe ein vertikaler Druck auf jede Seite der Wunde aufgebracht werden soll, sobald die Streifenelemente fixiert werden. Diese Röhrchen erfüllen somit eine Art Abstandhalterfunktion, um einen Kontakt der Streifenelemente mit der Wunde zu verhindern oder zu reduzieren.

Weitere Wundverschlüsse nach Art eines Klettverschlusssystems sind auch in der US 2004/193216 A1, der WO 2008/143689 A1, der WO 02/38096 A2 und der EP 1 462 075 A1 beschrieben. Im Ergebnis geht der Offenbarungsgehalt dieser Druckschriften nicht über den Inhalt der bereits beschriebenen Druckschriften hinaus.

Weiter ist in der US-6,176,868 B1 ein Wundverschlusssystem patentiert, das mindestens zwei längliche Elemente umfasst, die mit Hilfe von Zugmitteln in Richtung der Wunde gezogen werden, so dass eine Annäherung der beiden Wundränder aneinander erfolgt.

Die US-5,009,663-A offenbart eine weitere Methode zum Wundverschluss. Wesentliches Element des dort beschriebenen Verfahrens ist das knotenfreie Vernähen einer Wunde mit Hilfe von Plastikteilen.

Schließlich soll hier noch die DE 20 2006 015 861 U1 erwähnt werden, in der ebenfalls ein Wundverschluss offenbart ist, der auf dem Prinzip des Klettverschlusses basiert. Hier werden ebenfalls zwei an ihrer Unterseite mit einem Haftfilm beschichtete Hakenbänder beidseitig parallel zu den Wundrändern auf die Haut aufgeklebt. Die Fixierung der Wundränder und damit der Wundverschluss erfolgt hier mit Hilfe einzelner hantelförmiger Haftkörper, die auf den beiden Hakenbändern befestigbar sind.

Zusammenfassend kann festgestellt werden, dass aufgrund von Nachteilen der bisher bekannten Wundverschlüsse immer noch Bedarf an neuen, insbesondere (nachträglich) verstellbaren oder justierbaren Wundverschlüssen besteht. Ein Bedarf für derartige Wundverschlüsse besteht unter anderem in der Vermeidung eines Kompartmentsyndroms nach Unterarm- oder Unterschenkelfrakturen. Ein geeigneter Wundverschluss soll hierbei eine Traumatisierung von Nerven infolge von Stauungen innerhalb der Faszien, die häufig durch bakterielle Entzündungen insbesondere nach Unfällen und Kontaminationen der Wunde ausgelöst werden können, verhindern. Weiter birgt ein Wundverschluss mit Hilfe von Nahtmaterial das Risiko von Nadelstichverletzungen mit der damit verbundenen Narbenbildung. Für größere Wunden sind erhebliche Mengen an Nahtmaterial erforderlich. Schließlich ist die Infektionsgefahr des Personals durch Patientenblut zu erwähnen, sowie die bereits erwähnte Gefahr der Ausbildung eines Kompartmentsyndroms infolge einer Infektion für den Patienten.

Klammersysteme sind häufig aufgrund des dafür verwendeten metallischen Materials nicht für die Magnetresonanz und die Computertomographie tauglich. Außerdem ist die Gefahr der Narbenbildung durch das Klammern beträchtlich.

Kleber sind, sofern sie aufgrund der vergleichsweise geringen Zugfestigkeit des Wundverschlusses überhaupt einsetzbar sind, schlecht geeignet für nässende Wunden. Dies gilt auch für Wundpflaster, die generell nur topisch verwendet werden können.

Bei den reversiblen Verschlusstechniken, insbesondere den geschilderten Beispielen, kann in der Regel zwischen zwei Varianten unterschieden werden. Zum einen gibt es Ausführungen, die vergleichsweise schnell und einfach anzuwenden, dann aber in der Regel auf geradlinige Wundverläufe beschränkt sind. Zum anderen gibt es Wundverschlüsse, die auch für komplexere Wundverläufe einsetzbar sind, dann aber in der Regel zu einer zeitaufwändigen und vergleichsweise komplizierten Anwendung führen.

Die Erfindung stellt sich dementsprechend die Aufgabe, einen neuen Wundverschluss bzw. ein neues Wundverschlusssystem zur Verfügung zu stellen, das die geschilderten Nachteile deutlich reduziert. So soll es den Anwender, d.h. in der Regel den Arzt bzw. den Operateur in die Lage versetzen, eine Wunde vergleichsweise schnell zu verschließen. Zu diesem Zweck soll ihm ein überschaubares, einfach handhabbares System zur Verfügung gestellt werden. Zum anderen soll der neue Wundverschluss jedoch nicht nur für einfache, d.h. in der Regel geradlinige Wundverläufe geeignet sein, sondern auch für kompliziertere Wundverläufe, d.h. beispielsweise schräge oder geschwungene Wundverläufe. Weiter sollen die für den erfindungsgemäßen Wundverschluss eingesetzten Materialien und Bestandteile möglichst kostengünstig sein, um die Kosten insgesamt möglichst niedrig zu halten. Idealerweise soll hier auf bereits bekannte Materialien und Bestandteile zurückgegriffen werden können. Schließlich soll es mit Hilfe der Erfindung möglich sein, das Eindringen von Flüssigkeiten wie Wundflüssigkeit, Blut o. dgl. aus der Wunde heraus in die Hakenstruktur des Halteelements zu reduzieren oder sogar weitgehend zu verhindern.

Diese Aufgabe wird gelöst durch den Wundverschluss mit den Merkmalen des Anspruchs 1. Bevorzugte Ausführungen des erfindungsgemäßen Wundverschlusses sind in den abhängigen Ansprüchen 2 bis 15 dargestellt.
Der Wortlaut sämtlicher Ansprüche wird hiermit durch Bezugnahme zum Inhalt dieser Beschreibung gemacht.

Der erfindungsgemäße Wundverschluss ist (nachträglich) verstellbar bzw. anpassbar und zum Verschließen von Wunden vorgesehen. Er besitzt zum einen mindestens ein band- oder streifenartiges Halteelement, das zur Anbringung im Bereich mindestens eines Randes einer Wunde vorgesehen ist. Das Halteelement weist auf mindestens einem Teil mindestens einer seiner beiden Seiten eine Vielzahl von hakenartigen Elementen auf. Zum anderen umfasst der Wundverschluss mindestens ein netzartiges Abdeckelement, das zum im Wesentlichen vollständigen Abdecken der Wunde und der mit dem Halteelement versehenen Randbereiche vorgesehen ist.
Erfindungsgemäß sind die hakenartigen Elemente des Halteelements und die im Abdeckelement vorhandenen Maschen derart ausgebildet und/oder angeordnet, dass die Wunde durch ein formschlüssiges Eingreifen mindestens eines Teils der hakenartigen Elemente in die Maschen reversibel verschließbar ist.
Reversibel heißt, dass hakenartige Elemente und Maschen ohne Beschädigung wieder voneinander getrennt und die Vorgänge des Verschließens und Öffnens vielfach wiederholbar sind. Insofern kann der erfindungsgemäße Wundverschluss auch als "reversibler" Wundverschluss bezeichnet werden.

Schließlich weist das Halteelement bei der Erfindung an mindestens einem seiner beiden in Längsrichtung verlaufenden Ränder (d.h. denjenigen Rändern, die an die Wunde gegebenenfalls angrenzen) flüssigkeitsrückhaltende und/oder flüssigkeitsabweisende Mittel auf. Mit Hilfe solcher Mittel wird ein Eindringen von Wundflüssigkeit, Blut oder dergleichen aus der Wunde in die Hakenstruktur des Halteelements reduziert oder verhindert.
Eine solche Maßnahme ist deshalb von großem Vorteil, da die Hakenstruktur des Halteelements viele Hohlräume aufweist, in denen sich gegebenenfalls Wundflüssigkeit, Blut und dergleichen ansammeln und festsetzen können. Dies kann zu Problemen bei der Wundheilung führen, beispielsweise durch das Einschleppen und die Vermehrung von Keimen.

Solche flüssigkeitsrückhaltenden und/oder flüssigkeitsabweisenden Mittel können in der unterschiedlichsten Art und Weise realisiert werden. Insbesondere kann es sich hier um eine Schicht oder um eine Beschichtung handeln, die als eine Art Sperrschicht oder Sperrbeschichtung das Eindringen von Wundflüssigkeit, Blut oder dergleichen in die Hakenstruktur des Halteelements reduziert oder verhindert. Diese Schicht oder Beschichtung ist dann (mindestens) an dem Rand des Halteelements vorgesehen, der dem Rand der Wunde unmittelbar benachbart ist.

Vorzugsweise kann die genannte Schicht oder Beschichtung in Form eines Randwulstes ausgebildet sein, der zusätzlich aufgrund seiner Form eine (ab)dichtende Funktion ausübt. Insbesondere ist es zusätzlich oder alternativ möglich, die Schicht oder Beschichtung aus einem Schaumstoffmaterial bereitzustellen. Schaumstoffe sind bekanntlich künstlich hergestellte Stoffe mit zelliger Struktur und niedriger Dichte, die aus einer Vielzahl von Kunststoffen herstellbar sind. Solche Schaumstoffmaterialien können die genannten Flüssigkeiten, die aus einer Wunde austreten können, nicht nur aufhalten sondern ggf. auch aufnehmen und somit von der Hakenstruktur des Halteelements fernhalten.

Wie bereits erwähnt können die Schichten oder Beschichtungen, die als flüssigkeitsrückhaltende und/oder flüssigkeitsabweisende Mittel zum Einsatz kommen, vorzugsweise aus Kunststoffen bestehen. Hier können neben Polyurethan und Polyethylen vorzugsweise die Materialien zum Einsatz kommen, die für die Beschichtung oder Folie beim netzartigen Abdeckelement erwähnt wurden. Auf die entsprechenden Ausführungen wird Bezug genommen und verwiesen.

Als Schaumstoffe können vorzugsweise Polyethylenschaumstoffe, insbesondere Polyethylenschaumstreifen zum Einsatz kommen. Solche Schaumstoffstreifen sind einige Millimeter breit und bis zum einigen Millimetern hoch und besitzen eine Aufnahmekapazität für Flüssigkeiten wie Wasser von ohne weiteres bis zu 5 Vol.%. Solche Streifen können erfindungsgemäß an mindestens einem (Längs-)Rand des Halteelements oder an einem diesem Rand benachbarten Bereich der Unterseite des Halteelements befestigt, insbesondere angeklebt sein.

Unter Wundverschluss soll hier jede erfindungsgemäße Vorrichtung verstanden werden, bei der die Wundränder mindestens teilweise einander angenähert (adaptiert) werden und bei der die Wunde auf die beschriebene Weise verschlossen wird. Dies unterscheidet den erfindungsgemäßen Wundverschluss von anderen Arten des Wundverschlusses wie Nähen, Klammern oder Kleben. Unter "verstellbar" soll verstanden werden, dass der Wundverschluss nach seinem ersten Anbringen während des Zeitraums der Wundheilung verändert, d.h. an den jeweiligen Wundheilungszustand angepasst werden kann. Dies ist insbesondere während der ersten Tage nach Anbringen des Wundverschlusses von Bedeutung. Dementsprechend kann der erfindungsgemäße Wundverschluss nach seinem ersten Anbringen wieder geöffnet und gegebenenfalls wieder angebracht werden. Dies ermöglicht zum einen eine Kontrolle der Wunde und eine Anpassung des Verschlusses je nach Verlauf der Wundheilung.

Erfindungsgemäß kann der erfindungsgemäße Wundverschluss eine zusätzliche Folie umfassen, die nach dem Verschließen der Wunde über dem Wundverschluss anordenbar ist. Es handelt sich also hier nicht um eine mit dem netzartigen Abdeckelement von vorneherein verbundene Folie, sondern um eine Folie, die nachträglich über dem Wundverschluss, und dabei zumindest über dem Abdeckelement angeordnet werden kann.

Diese zusätzliche Folie dient dazu, die durch die Maschenstruktur des netzartigen Abdeckelements noch freiliegenden Wundbereiche zu verschließen. Hier kann es sich bei der Folie vorzugsweise um eine Kunststofffolie, insbesondere um eine Folie aus Polyurethan handeln. Polyurethan und gegebenenfalls andere geeignete Kunststoffmaterialien sind gas- und wasserdampfdurchlässig, so dass ein entsprechender Stoffaustausch zwischen Wunde und Umgebung ermöglicht wird. Es ist dadurch eine Feuchtigkeitsregulation durch die Dicke und chemische Zusammensetzung der Folie einstellbar, die anderweitig nur durch Verwendung von Hydrokolloidpartikeln, wie beispielsweise im registrierten Produkt Urgotül®, die in seltenen Fällen Allergien hervorrufen können, erreichbar wäre.

Die über dem Wundverschluss anordenbare zusätzliche Folie kann an Randbereichen, die beim bestimmungsgemäßen Gebrauch der Folie über die Abmessungen des Wundverschlusses (einschließlich seiner Befestigungsbereiche bei den Halteelementen) hinausragen, Befestigungsmittel für die Festlegung an der Haut oder dem sonstigen Gewebe aufweisen. Hier kann es sich insbesondere um Klebemittel wie Klebestreifen und dergleichen handeln. Auf diese Weise wird gewährleistet, dass die Abdeckung des eigentlichen Wundverschlusses durch die Folie dauerhaft erhalten bleibt. Die Befestigungsmittel, vorzugsweise Klebemittel sind vorzugsweise reversibel an der Haut bzw. dem Gewebe anbringbar, um ein späteres Entfernen der Folie auf einfache Weise zu gewährleisten.

Das erfindungsgemäß eingesetzte Halteelement ist bei der Erfindung vorzugsweise aus Kunststoff gefertigt. Insbesondere handelt es sich hierbei um einen biokompatiblen Kunststoff. Biokompatibel bedeutet, dass das entsprechende Material keinen negativen Einfluss auf den Organismus ausübt, der mit dem erfindungsgemäßen Wundverschluss versorgt wird.

Als Kunststoffmaterialien für das Halteelement kommen grundsätzlich die unterschiedlichsten Kunststoffe in Frage. Zu nennen sind hier insbesondere Polypropylen (PP), Polystyrol (PS), Acrylnitrilbutadienstyrol (ABS), Polyester (PES) oder Polyamide (PA).

Das genannte Halteelement kann grundsätzlich in unterschiedlichster Weise am Wundrand festlegbar sein, beispielsweise durch Verankerungsmittel, mit deren Hilfe das Halteelement an der Haut oder dem sonstigen Gewebe festlegbar ist. Vorzugsweise weist das Halteelement jedoch auf der den hakenartigen Elementen abgewandten Seite Klebemittel zur Anbringung des Halteelements am Wundrand auf. Insbesondere handelt es sich dabei um eine, vorzugsweise auf die gesamte entsprechende Fläche aufgebrachte Klebeschicht.
Die Klebemittel, insbesondere die Klebeschicht sind dabei vorzugsweise so ausgebildet, dass das Halteelement nach seiner bestimmungsgemäßen Verwendung wieder von der Haut oder dem entsprechenden Gewebe abgezogen werden kann. Dies wird optional durch eine spitz auslaufende Form der Halteelemente begünstigt.

Bei den verwendeten Klebemitteln kann es sich um alle möglichen biokompatiblen, insbesondere haut- bzw. gewebeverträglichen Klebstoffe handeln. Vorzugsweise bestehen solche Klebemittel, insbesondere die Klebeschicht, aus Alkoxy- oder Alkylacrylaten oder aus Polymeren von Butadien- oder Isopren-basierten Kautschuken. Hier sind auch übliche Copolymere solcher Klebstoffe einsetzbar.

Die Länge der band- oder streifenartigen Halteelemente ist grundsätzlich frei wählbar. So können solche Halteelemente speziell für bestimmte Wundarten und Wundlängen bereitgestellt werden, beispielsweise in Form von Bändern oder Streifen zwischen 1 cm Länge und 20 cm Länge. Vorzugsweise werden jedoch die Halteelemente in Form längerer Bänder oder Streifen bereitgestellt, beispielsweise auch in Form von Rollen, von denen dann das entsprechende Halteelement in der gewünschten Länge abgelängt werden kann.

Auch Breite und Höhe des Halteelements (letzteres einschließlich der hakenartigen Elemente) sind frei wählbar. Es sind jedoch zum einen Breiten von weniger als 20 mm, insbesondere Breiten zwischen 5 mm und 10 mm hervorzuheben. Bezüglich der Höhe sind Werte zwischen 0,5 mm und 5 mm bevorzugt, wobei insbesondere Werte zwischen 0,5 mm und 1,5 mm in Frage kommen.

Bezüglich der Höhe und insbesondere auch bezüglich der Breite ist zu berücksichtigen, dass das Halteelement umso flexibler, d.h. biegsamer ausgestaltet werden kann, je geringer seine Höhe und insbesondere je geringer seine Breite ist. Solche schmalen und gegebenenfalls auch niedrigen Halteelemente mit hoher Flexibilität lassen sich besonders gut an nicht-geradlinige Wundverläufe wie schräge oder geschwungene Wundverläufe anpassen und anlegen.

Die hakenartigen Elemente des Halteelements, die dann in Maschen des netzartigen Abdeckelements eingreifen, können bei der Erfindung grundsätzlich in beliebiger Weise ausgestaltet sein. Voraussetzung ist nur die erwähnte hakenartige Struktur, die das Hintergreifen der hakenartigen Elemente in die die Maschen begrenzenden Fäden gewährleistet.

Vorzugsweise sind die hakenartigen Elemente des Halteelements bei der Erfindung pilzkopfförmig ausgebildet. Hier werden dann also die Fäden der Maschen unterhalb des Pilzkopfes an der dort vorhandenen "hakenartigen" Struktur festgelegt.

In Weiterbildung können die hakenartigen Elemente des Halteelements Widerhaken aufweisen. Hier handelt es sich um (meist zusätzliche) Haken, deren Spitze der Richtung des Haupthakens entgegengesetzt ist. Dadurch wird eine besonders gute Verankerung und Festlegung erreicht.

Insbesondere sind solche Widerhaken bei der Erfindung harpunenförmig ausgebildet. Solche Formen sind insbesondere aus der Fischjagd bekannt.

Unter "netzartigem" Abdeckelement soll bei der Erfindung jedes Abdeckelement verstanden werden, bei dem mit Hilfe von fadenartigen Materialien eine Netzstruktur aufgebaut ist. Diese Netzstruktur besitzt erfindungsgemäß eine Elastizität, so dass die Größe der Maschen des Netzes durch Zug (reversibel) vergrößert bzw. verkleinert werden kann. Hier kann es sich insbesondere um Gewirke oder um Gewebe handeln.

Vorzugsweise handelt es sich bei dem netzartigen Abdeckelement bei der Erfindung um ein Gewebe, wobei es sich insbesondere um sogenannten Tüll handelt. Die netzartige Struktur beim Tüll entsteht, indem man beim Weben je zwei beisammen liegende Kettfäden nach jedem Einschuss verdreht. Entsprechende Gewebe werden unter anderem für Gardinen verwendet.

Grundsätzlich ist es bevorzugt, wenn das netzartige Abdeckelement aus Kunststofffäden, insbesondere aus nicht resorbierbaren Kunststofffäden gefertigt ist. Solche Kunststofffäden können insbesondere aus Polyester, Polyamid oder Polypropylen bestehen. Hervorzuheben ist auch insbesondere das Material Elastan, ein Block-Copolymer aus den Bestandteilen Polyurethan und Polyethylenglykol. Dieses Material kann mit anderen Materialien wie Polyamiden, Polypropylen oder Polyester gemischt (geblendet) sein.

In Weiterbildung hat das netzartige Abdeckelement vorzugsweise ein Gewicht zwischen 10 und 100 g/m². Innerhalb dieses Bereichs sind Gewichte zwischen 15 und 80 g/m² bevorzugt.

Insbesondere besitzt das netzartige Abdeckelement eine Reißkraftdehnung zwischen 30 % und 100 %, vorzugsweise zwischen 30 % und 60 %. Damit können in der Regel die üblichen Spannungen, die beim Verschließen einer Wunde auftreten ohne Weiteres abgefangen werden. Die Reißkraftdehnung oder Reißdehnung ist der Materialkennwert, der die aufgetretene Verlängerung einer Probe beim Reißen, bezogen auf die Anfangsmesslänge, angibt.

Bei bevorzugten Ausführungsformen der Erfindung ist das netzartige Abdeckelement an mindestens einem seiner Ränder so ausgebildet, dass dort mindestens ein flächiges oder aus einzelnen oder mehreren Fäden gebildetes Mittel zur Ausrichtung des Abdeckelements in Bezug auf die Wunde und in Bezug auf das Halteelement vorgesehen ist. Durch solche Mittel wird die Ausrichtbarkeit und damit auch die Verstellbarkeit des Wundverschlusses insgesamt erleichtert.
So können beispielsweise an einem Rand oder an mehreren Rändern des Abdeckelements einzelne Fäden, mehrere Fäden oder flächige Bereiche, welche vorzugsweise schmal sind, vorgesehen sein ("überstehen"). Mit Hilfe dieser Mittel kann dann das Abdeckelement in Bezug auf die Wunde und in Bezug auf das Halteelement ausgerichtet und auch gespannt werden. In diesem durch die Mittel definierten Zustand wird dann das Abdeckelement durch Eingriff der Maschen in die hakenartigen Elemente des Halteelements festgelegt und die Wunde in der gewünschten Weise verschlossen.
Solche Ausrichtmittel oder auch Zugmittel am Abdeckelement erleichtern auch ein später gegebenenfalls vorgenommenes Trennen des Abdeckelements von den Halteelementen, da der Anwender das Abdeckelement mit Hilfe solcher Mittel in einfacher Weise, beispielsweise sukzessive, von den Haltemitteln (wieder) abziehen kann. Damit wird die Wunde wieder (sukzessive) freigelegt und es kann die gegebenenfalls notwendige Verstellung/Justierung/Anpassung des Wundverschlusses an den Prozess der Wundheilung vorgenommen werden.

In Weiterbildung ist das netzartige Abdeckelement optional auf mindestens einer seiner beiden Seiten, d.h. auf seiner Oberseite und/oder seiner Unterseite, mindestens teilweise mit einer Beschichtung oder Folie versehen. Auf diese Weise kann über einem Teil der Wunde oder über der gesamten Wunde eine geschlossene Abdeckung bereitgestellt werden. Bei dem netzartigen Abdeckelement, das die Wunde erfindungsgemäß im wesentlichen vollständig abdeckt, liegen ja diejenigen Teile der Wunde offen, die von den offenen Teilen der Maschen überdeckt werden.

Die genannte Beschichtung oder Folie ist vorzugsweise an den Bereichen des netzartigen Abdeckelements, die zum Zusammenwirken mit den hakenartigen Elementen des Halteelements vorgesehen sind, nicht vorhanden. Auf diese Weise wird gewährleistet, dass die Maschen an diesen Stellen ohne Beschichtung/Folie weiterhin offen sind und so mit den hakenartigen Elementen zusammenwirken können.

Bei den beschriebenen Ausführungsformen mit Beschichtung oder Folie ist die Maschenstruktur des netzartigen Abdeckelements mit der Beschichtung oder Folie verbunden, so dass ein einheitliches Abdeckelement entsteht, das als solches handhabbar ist. Ausführungen mit einer (zunächst) getrennten Folie wurden bereits beschrieben.

Die bei den bereits beschriebenen Ausführungsformen vorgesehene Beschichtung oder Folie ist vorzugsweise wasserdampfdurchlässig und insbesondere auch gasdurchlässig. Auf diese Weise ist ein Austausch von Wasser und Gas durch das Abdeckelement hindurch zwischen Wunde und Umgebung möglich, was für den Heilungsprozess förderlich ist.

Bei der Beschichtung oder Folie handelt es sich vorzugsweise um Beschichtungen oder Folien aus Kunststoff. Hervorzuheben ist hier Polyurethan als gas- und wasserdampfdurchlässiges Material. Bevorzugt in Frage kommen auch Polymere oder Copolymere mit polaren funktionellen Gruppen wie -OR oder -COOR, wie sie beispielsweise in Polysacchariden oder Polyalkoholen vorkommen. R steht für H oder Alkyl, insbesondere C₁-C₆-Alkyl.

Wie eingangs im Zusammenhang mit der Erfindung erwähnt erfolgt der Wundverschluss letzten Endes durch formschlüssiges Eingreifen mindestens eines Teils der hakenartigen Elemente des/der Halteelements/Halteelemente in die Maschen des Abdeckelements (d.h. in mindestens einen Teil der Maschen). Dementsprechend können Anzahl, Abmessungen und/oder Struktur der hakenartigen Elemente des Halteelements einerseits und der Maschen/Fäden des Abdeckelements andererseits in gewünschter Weise aufeinander abgestimmt sein. Beispielsweise kann durch Wahl der Anordnung und/oder Abmessungen der Hakenelemente und/oder durch Wahl der Maschengröße und/oder der Elastizität des Netzes die Anzahl der in eine Masche eingreifenden Hakenelemente festgelegt werden.

In diesem Zusammenhang ist es erfindungsgemäß bevorzugt, wenn die Maschenweite des netzartigen Abdeckelements mindestens doppelt so groß ist wie der Durchmesser der hakenartigen Elemente des Halteelements. Auf diese Weise wird gewährleistet, dass die hakenartigen Elemente in die Maschen hineinpassen und deshalb ein Zusammenwirken zwischen den Fäden, die die Maschen begrenzen, und der Hakenstruktur der Halteelemente in einfacher Weise erfolgen kann.

In diesem Zusammenhang ist es noch weiter bevorzugt, wenn die Maschenweite des netzartigen Abdeckelements zwei bis vier Mal so groß ist wie der Durchmesser der hakenartigen Elemente des Halteelements. Weiter sind Ausführungsformen der Erfindung hervorzuheben, bei denen die Anordnung der hakenartigen Elemente auf dem Halteelement derart gewählt ist, dass zwischen eins und drei hakenartige Elemente in eine Masche des netzartigen Abdeckelements passen. Auf diese Weise kann erreicht werden, dass häufig mehr wie ein hakenartiges Element in eine Masche eingreift und so nicht nur an einer Fadenbegrenzung einer Masche ein Zusammenwirken zwischen Faden und Hakenelement erfolgt.

Bei noch weiter bevorzugten Ausführungsformen für bestimmte Wundformen und -verläufe wird erfindungsgemäß gewährleistet, dass bezogen auf die Anordnung des Halteelements am Wundrand in Richtung des Verlaufs der Wunde ("längs des Wundverlaufs") und in Richtung der auf die Wunde aufzubringenden Wundspannung ("quer zum Wundverlauf) des netzartigen Abdeckelements unterschiedlich viele hakenartige Elemente des Halteelements in eine Masche passen. Auf diese Weise kann durch das Zusammenwirken von hakenartigen Elementen und Maschen gegebenenfalls in einer Richtung ein stärkerer Zug auf die Wunde aufgebracht werden als in der anderen Richtung. Insbesondere passen bei solchen Ausführungen in horizontaler Richtung ein hakenartiges Element in eine Masche und in vertikaler Richtung zwei hakenartige Elemente in eine Masche.

Bei Zugbelastung des Abdeckelementes können aufgrund der variablen und in diesem Falle kleiner werdenden Maschenweite durchschnittlich weniger hakenartige Elemente pro Masche eingreifen. Der dadurch gegebenenfalls bedingte Verlust der Haltewirkung der Hakenelemente in den Maschen wird jedoch dann durch die zusätzliche Spannung des Abdeckelementes kompensiert.

Im Zusammenhang mit den vorigen Ausführungen steht auch das bevorzugte Merkmal des erfindungsgemäßen Wundverschlusses, dass die Haltefestigkeit des Wundverschlusses an der Wunde bei Zugbelastung des netzartigen Abdeckelements senkrecht zum Wundverlauf, d.h. in horizontaler Richtung (s. oben) mindestens 10 N (Newton) beträgt. Vorzugsweise liegt die Zugbelastung zwischen 12 N und 20 N.

In Weiterbildung liegt der erfindungsgemäße Wundverschluss vorzugsweise in steriler Form vor. Insbesondere ist er in einer sterilen Verpackung angeordnet. Die sterile Verpackung gewährleistet, dass der Anwender, in der Regel der Arzt oder Operateur, den Wundverschluss nur aus der Verpackung entnehmen muss und sofort verwenden kann.

Die Erfindung kann auch Teil eines Kits zum Verschließen von Wunden sein. Dieser Kit umfasst erfindungsgemäß mindestens einen Wundverschluss wie er von der Erfindung definiert ist und beschrieben wurde. Dieser Kit umfasst den Wundverschluss in sterilisierter Form, vorzugsweise in einer sterilisierten Verpackung.

Die Erfindung ist in Kombination mit einer ganzen Reihe von Vorteilen verbunden. So wird ein Wundverschluss zur Verfügung gestellt, mit dem nicht nur unkomplizierte Wunden und Wundverläufe sondern auch kompliziertere Wunden und Wundverläufe schnell und flexibel versorgt werden können. Die bisherige Beschränkung bekannter Wundverschlüsse auf entweder gerade Wundverläufe mit vergleichsweise einfacher Versorgung oder auf kompliziertere Wundverläufe mit aufwändiger Versorgung entfällt. Das Prinzip der Wundversorgung ist bei der Erfindung immer dasselbe, unabhängig davon, welche Art von Wunde oder Wundverlauf versorgt wird. Der Anwender, d.h. in der Regel der Arzt oder Operateur hat immer die selben vergleichsweise einfach handhabbaren Bestandteile des erfindungsgemäßen Wundverschlusses vor sich und kann diese in der ihm vertrauten Weise anwenden.

Die Bestandteile des erfindungsgemäßen Wundverschlusses sind einfach zugänglich oder in einfacher Weise herstellbar. Es handelt sich um übliche Klettbänder, die vorzugsweise mit vergleichsweise geringen Breiten und Höhen zur Verfügung gestellt werden können. Bei der Wundabdeckung handelt es sich vorzugweise um ein einfaches Gewebe, wie insbesondere Tüll, das ebenfalls kostengünstig erhältlich ist. Die Materialien, aus denen die Bestandteile des erfindungsgemäßen Wundverschlusses gefertigt sind, d.h. in der Regel die Kunststoffmaterialien, können weitgehend frei gewählt und auf den Einsatz in der Medizintechnik abgestimmt werden. Aufgrund der Verwendung von Kunststoffmaterialien lässt sich in vergleichsweise einfacher Weise ein hoher Hygienestandard gewährleisten. Gegebenenfalls notwendige Modifikationen, beispielsweise entweder durch Verwendung einer zusätzlichen Folie zur Wundabdeckung oder durch vorherige Verbindung des Abdeckelements mit einer Folie oder Beschichtung, können in einfacher Weise realisiert werden.

Durch den nicht direkt in die Wunde eingreifenden Haltemechanismus in Verbindung mit den flüssigkeitsrückhaltenden bzw. flüssigkeitsabweisenden Mitteln in Nachbarschaft zu den Wundrändern wird die Wundheilung durch den erfindungsgemäßen Wundverschluss nicht beeinträchtigt. Dies reduziert das Infektionsrisiko beträchtlich und trägt zur Vermeidung von Narbenbildung bei. Dies ist besonders deshalb hervorzuheben, da ästhetische Gesichtspunkte bei der Wundversorgung eine immer größere Rolle spielen. Erwähnt werden soll auch, dass der erfindungsgemäße Wundverschluss gerade für sensitive Patienten und für Kinder eine besondere Erleichterung in der Wundversorgung darstellt.

Weitere Merkmale der Erfindung ergeben sich aus der nachfolgenden Beschreibung von bevorzugten Ausführungsformen in Verbindung mit den Beispielen und den Unteransprüchen. Hierbei können die einzelnen Merkmale jeweils für sich allein oder zu mehreren in Kombination miteinander verwirklicht sein. Die beschriebenen Ausführungsformen dienen lediglich zur Erläuterung und zum besseren Verständnis der Erfindung und sind in keiner Weise einschränkend zu verstehen.

In den Zeichnungen zeigen
- Figur 1:: eine erste Ausführungsform des erfindungsgemäßen Wundverschlusses in schematischer Darstellung (Draufsicht),
- Figur 2:: eine weitere Ausführungsform des erfindungsgemäßen Wundverschlusses in schematischer Darstellung (Draufsicht),
- Figur 3:: eine dritte Ausführungsform des erfindungsgemäßen Wundverschlusses in schematischer Darstellung (Schnittansicht),
- Figur 4:: das Halteelement nach der Erfindung mit flüssigkeitsabweisendem Mittel, und
- Figur 5a,b:: die schematische Darstellung des Verschließens einer Wunde mit Hilfe des erfindungsgemäßen Wundverschlusses.

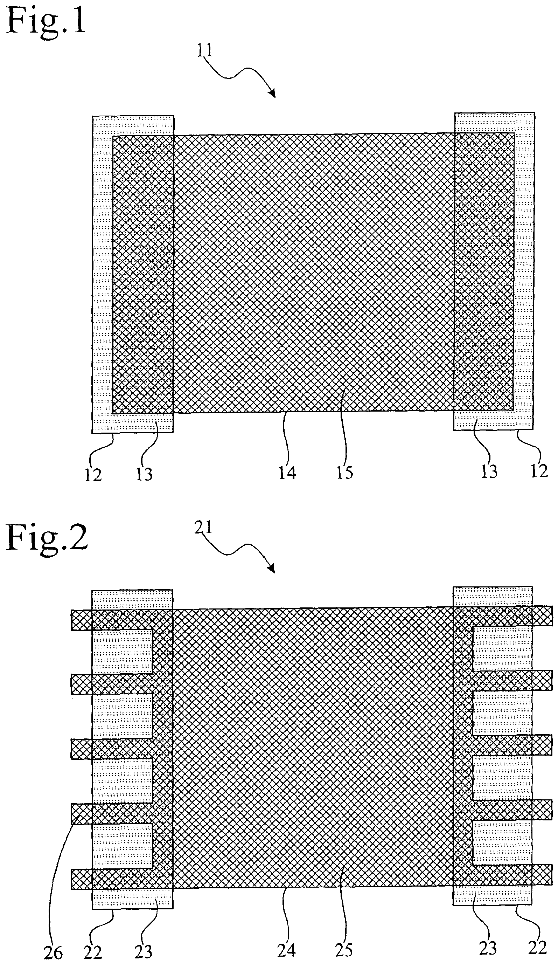

Figur 1 zeigt den erfindungsgemäßen Wundverschluss 11 in schematischer Draufsicht. Dieser Wundverschluss 11 besteht aus zwei band- bzw. streifenartigen Halteelementen 12, die auf ihrer dem Betrachter zugewandten Oberseite eine Vielzahl von hakenartigen Elementen 13 aufweisen. Auf ihren dem Betrachter abgewandten Seiten besitzen die Halteelemente 12 jeweils eine Klebeschicht, die (in Figur 1 nicht dargestellt) zur Befestigung der Halteelemente 12 auf dem die Wunde umgebenden Gewebe vorgesehen sind.

Schließlich zeigt Figur 1 das netzartige Abdeckelement 14 als Bestandteil des erfindungsgemäßen Wundverschlusses 11. Dieses Abdeckelement 14 besteht aus einem Gewebe mit einer Vielzahl von Maschen 15, die in Figur 1 zeichnerisch nur angedeutet sind. Diese Maschen 15 sind dazu vorgesehen mit den hakenartigen Elementen 13 der Halteelemente 12 zusammenzuwirken, um auf diese Weise das Verschließen der Wunde zu bewirken.

An dieser Stelle wird für alle Figuren darauf hingewiesen, dass die dort gewählten Darstellungen idealisiert sind. Dies betrifft insbesondere auch die Darstellungen der netzartigen Abdeckelemente (s. beispielsweise Abdeckelement 14 gemäß Figur 1). Es ist für den Fachmann beispiels weise klar, dass die in den Figuren dargestellten Maschen der Abdeckelemente (s. zum Beispiel Maschen 15 des Abdeckelements 14) über das gesamte Abdeckelement nicht gleichförmig sind, sondern je nach dem für den Wundverschluß auf die Wunde aufgebrachten Zug, der wiederum in unterschiedlichen Richtungen unterschiedlich groß sein kann, eine unterschiedliche Größe aufweisen. Dies wurde aus Gründen der Übersichtlichkeit in den Figuren nicht dargestellt.

Wie Figur 1 zeigt, wird durch das Abdeckelement 14 eine zwischen den Halteelementen 12 vorhandene Wunde vollständig abgedeckt. Diese Abdeckung umfasst auch die Randbereiche der Wunde und einen Teil der Oberfläche der Halteelemente 12.

Figur 2 zeigt eine weitere Ausführungsform 21 des erfindungsgemäßen Wundverschlusses. Auch hier sind zwei band- oder streifenartige Halteelemente 22 vorgesehen, die auf ihrer dem Betrachter zugewandten Oberseite eine Vielzahl von hakenartigen Elementen 23 aufweisen. Auch hier ist auf den Unterseiten der Halteelemente 22 eine nicht dargestellte Klebeschicht zur Befestigung der Halteelemente 22 auf dem die Wunde umgebenden Gewebe vorgesehen.

Weiter umfasst der Wundverschluss 21 gemäß Figur 2 das netzartige Abdeckelement 24, das aus einem Gewebe mit Maschen 25 besteht. Dieses Abdeckelement 24 gemäß Figur 2 ist so ausgestaltet, dass es an zwei seiner gegenüberliegenden Ränder schmale Flächenbereiche 26 aufweist, die zur Ausrichtung des Abdeckelements 24 in Bezug auf die Wunde und in Bezug auf die Halteelemente 22 vorgesehen sind. Diese Flächenbereiche können auch als Verlängerungen 26 des Abdeckelements 24 betrachtet werden. Wie in der Beschreibung bereits erläutert, können diese Flächenbereiche (auch im Sinne von Zugmitteln) dazu dienen, das Abdeckelement 24 über der Wunde zu verspannen und dann an den Halteelementen 22 festzulegen. Die Flächenbereiche 26 erleichtern auch das Abziehen des Abdeckelements 24 von den Halteelementen 22, wenn der Bereich der Wunde wieder geöffnet und von oben zugänglich gemacht werden soll. In diesem Zusammenhang kann es von Vorteil sein, wenn die Flächenbereiche 26 über die Halteelemente 22 hinausragen wie dies in Figur 2 dargestellt ist. Auf diese Weise können nämlich die Flächenbereiche 26 in einfacher Weise vom Anwender gefasst und gehandhabt werden.

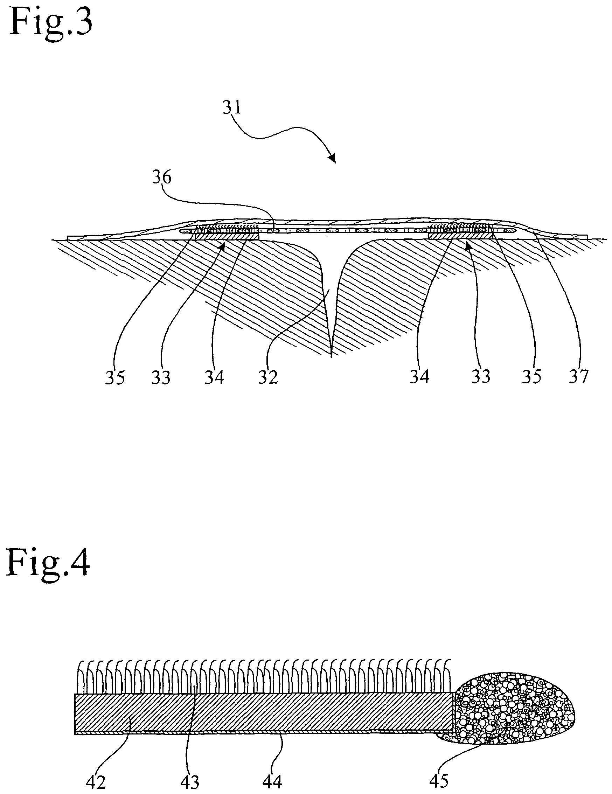

Figur 3 zeigt einen erfindungsgemäßen Wundverschluss 31, der über einer Wunde angebracht und zusätzlich mit einer den Wundverschluss überdeckenden (randseitig verklebten) Folie versehen ist. Diese Anordnung ist in Figur 3 in einer schematischen Schnittansicht dargestellt.

Figur 3 zeigt einerseits die Wunde 32 mit dem sie umgebenden Gewebebereich.

Zu beiden Seiten der Wunde 32 sind band- oder streifenartige Halteelemente 33 angebracht, die im Fall der Figur 3 senkrecht zur Zeichenebene verlaufen. Diese Halteelemente 33 besitzen an ihrer Unterseite Klebeschichten 34, mit deren Hilfe sie auf dem Gewebe (reversibel) festgelegt sind. An ihren Oberseiten weisen die Halteelemente 33 eine Vielzahl von hakenartigen Elementen 35 auf.

Weiter zeigt Figur 3 das netzartige Abdeckelement 36, das von einem Gewebe gebildet ist und eine Vielzahl von in Figur 3 nicht näher dargestellten Maschen aufweist. Dieses Abdeckelement 36 ist gemäß Figur 3 so angeordnet, dass die Wunde 32 durch ein formschlüssiges Eingreifen mindestens eines Teils der hakenartigen Elemente 35 der Halteelemente 33 in die Maschen des Abdeckelements 36 verschließbar ist. Dieses Verschließen erfolgt in der Regel in der Art, dass das Abdeckelement 36 mit einem der beiden Halteelemente verbunden wird und dann die beiden Ränder der Wunde 32 durch Zug aneinander angenähert (adaptiert) werden. Dann wird das Abdeckelement 36 auch mit dem zweiten Halteelement 33 verbunden. Das Verspannen der Wundränder gegeneinander kann durch externe Hilfsmittel temporär unterstützt werden.

Schließlich zeigt Figur 3 eine weitere Folie 37, die oberhalb des Wundverschlusses 31 aus Halteelementen 33 und Abdeckelement 36 angeordnet ist. Diese Folie 37 dient, wie in der Beschreibung erläutert, dazu, die Wunde vollständig (keimdicht) gegen die Umgebung abzudecken. Wie ebenfalls bereits erwähnt kann es sich hier um eine gas- und/oder wasserdampfdurchlässige Folie, beispielsweise aus Polyurethan handeln, um einen Stoffaustausch zwischen Wunde und Umgebung zu gewährleisten. Die Folie 37 ist an ihren Randbereichen mit dem die Wunde umgebenden Gewebe verklebt.

In Figur 4 ist ein band- oder streifenartiges Halteelement, das erfindungsgemäß einsetzbar ist, dargestellt. Dieses Halteelement 42 dient dazu, das Eindringen von Wundflüssigkeit, Blut oder dergleichen aus der Wunde in die Hakenstruktur des Halteelements zu reduzieren oder zu verhindern. Zu diesem Zweck weist das Halteelement neben den hakenartigen Elementen 43 an seiner Oberseite und gegebenenfalls einer Klebeschicht 44 an seiner Unterseite an mindestens einem seiner beiden (längeren) Ränder ein flüssigkeitsrückhaltendes und flüssigkeitsabweisendes Mittel in Form eines wulstartigen Schaumstoffstreifens 45 auf. Dieser Schaumstoffstreifen 45, der auch an beiden Rändern des Halteelements 42 vorgesehen sein kann, besteht vorzugsweise aus Polyethylen. Der Schaumstoffstreifen 45 ist vorzugsweise an dem entsprechenden Randbereich des Halteelements 42 und/oder im Bereich der Unterseite des Halteelements 42 angeklebt. Bei seiner bestimmungsgemäßen Verwendung beim Verschließen einer Wunde wird das Halteelement 42 derart am Rand der Wunde befestigt, dass der Schaumstoffstreifen 45 dem Wundrand zugewandt ist.

Im vorliegenden Fall erfüllt der Schaumstoffstreifen 45 zwei Funktionen. Zum einen hält er durch seine Ausgestaltung als Wulst die Wundflüssigkeit vom Halteelement 42 und damit von den hakenartigen Elementen 43 ab. Er erfüllt also somit eine (ab-)dichtende Funktion. Zum anderen ist der Streifen 45 aufgrund des gewählten Materials (Schaumstoff) in der Lage Wundflüssigkeit aufzunehmen, so dass die zuvor genannte dichtende Funktion unterstützt wird. All dies verhindert, dass Wundflüssigkeit in die Hakenstruktur des Halteelements 42 eindringen kann. Damit werden die in der Beschreibung bereits diskutierten Hygieneprobleme bei der Wundheilung verhindert oder doch zumindest reduziert.

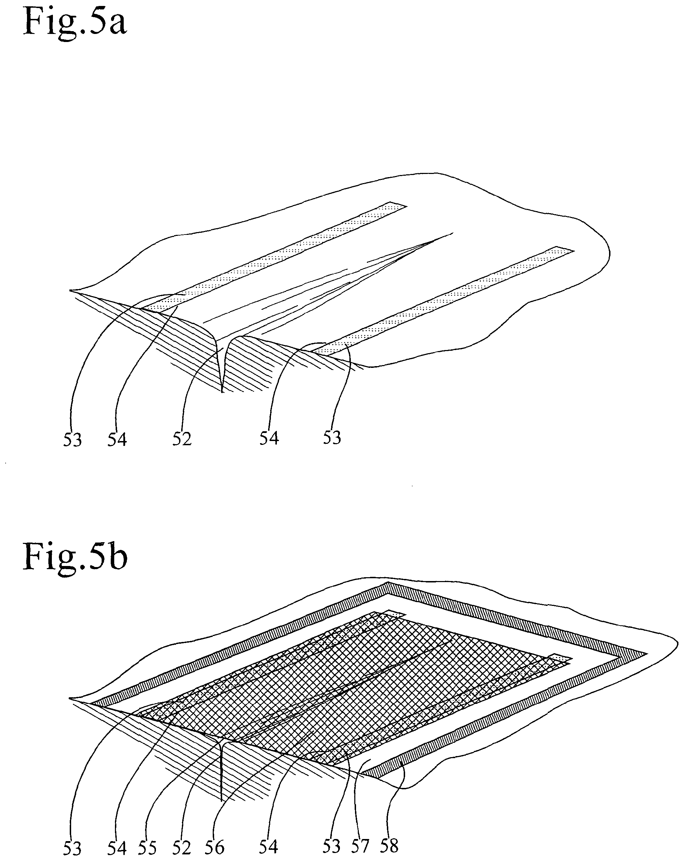

Figur 5 zeigt schließlich das Verschließen einer Wunde, wie sie mit Hilfe des erfindungsgemäßen Wundverschlusses vorgenommen werden kann.

Dazu werden in einem ersten Stadium an der Wunde 52, die im dargestellten Fall einen länglichen, gekrümmten Verlauf besitzt, zu beiden Seiten jeweils ein Halteelement 53 angebracht. Die schematische Darstellung der Figur 5 zeigt, dass die Halteelemente 53 parallel zueinander und geradlinig ausgerichtet sind. Die über den Wundverlauf inhomogen auftretende Wundspannung wird dann durch die ungleichmäßige Maschenweite im Abdeckelement 55 ausgeglichen.
Die Halteelemente 53 besitzen auf ihrer dem Betrachter zugewandten Oberseite die bereits beschriebenen hakenartigen Elemente 54.

Dieses erste Verfahrensstadium ist in Figur 5a verdeutlicht.

Figur 5b zeigt ein späteres Stadium des Verschließens der Wunde 52, bei dem der Wundverschluss im wesentlichen vervollständigt ist. Die Wunde ist weitgehend verschlossen und zwar sind durch die gewählte Anordnung die Maschen 56 des netzartigen Abdeckelements 55 mit den hakenartigen Elementen 54 formschlüssig im Eingriff, so dass die Ränder der Wunde 52 gegeneinander verspannt bleiben.

Wie bereits geschildert kann der Wundverschluss so erfolgen, dass das Abdeckelement 55 zunächst an einem Halteelement 53 auf einer Seite befestigt wird und dann die Wunde unter Zug verschlossen wird. Dabei werden die Maschen 56 des als Netz vorliegenden Abdeckelements 55 gedehnt und aufgeweitet, wobei die dafür notwendige Elastizität durch das für die Ausbildung des Netzes verwendete Fadenmaterial und/oder die Konstruktion des Netzes bereitsgestellt wird. Dann wird das Abdeckelement 55 mit dem Halteelement 53 der anderen Seite verbunden. Dies kann durch externe Hilfsmittel, die der Anwender zum Zusammenziehen der Wunde kurzfristig einsetzt, übernommen oder unterstützt werden.

Über das Abdeckelement 55 wird, um die Wunde vor externen Kontaminationen (Wasser, Partikel, Keime) zu schützen und eine Feuchtigkeitsregulation zu bewirken, eine gasdurchlässige Folie 57 an ihren Randbereichen 58 aufgeklebt, welche entfernt werden kann, ohne das Abdeckelement 55 hierbei zu lockern.

Figur 5b zeigt auch, dass die Wunde aufgrund des maschenartigen Aufbaus des Abdeckelements von oben zumindest teilweise sichtbar bleibt, so dass die Wundheilung kontrolliert werden kann. Der Wundverschluss kann abhängig vom Wundverlauf jederzeit geöffnet und gegebenenfalls wieder verschlossen werden, so dass ein verstellbarer/justierbarer/anpassbarer Wundverschluss gegeben ist.

## Patentansprüche

1. Verstellbarer Wundverschluss (11; 21; 31) zum Verschließen von Wunden, mit
- mindestens einem band- oder streifenartigen Halteelement (12; 22; 33), das zur Anbringung im Bereich mindestens eines Randes einer Wunde vorgesehen ist, wobei das Halteelement (12; 22; 33) auf mindestens einem Teil mindestens einer seiner beiden Seiten eine Vielzahl von hakenartigen Elementen (13; 23; 35) aufweist, und
- mindestens einem netzartigen Abdeckelement (14; 24; 36), das zum im wesentlichen vollständigen Abdecken der Wunde und der mit dem Halteelement versehenen Randbereiche vorgesehen ist,
- wobei die hakenartigen Elemente (13; 23; 35) des Halteelements (12; 22; 33) und die Maschen (15; 25) des Abdeckelements (14; 24; 36) derart ausgebildet und angeordnet sind, dass die Wunde durch ein formschlüssiges Eingreifen mindestens eines Teils der hakenartigen Elemente in die Maschen reversibel verschließbar ist, und
- wobei das Halteelement (42) an mindestens einem seiner beiden in Längsrichtung verlaufenden Ränder flüssigkeitsrückhaltende und/oder flüssigkeitsabweisende Mittel (45) aufweist, mit deren Hilfe ein Eindringen von Wundflüssigkeit, Blut oder dergleichen aus der Wunde in die Hakenstruktur des Halteelements reduzierbar oder verhinderbar ist.

2. Wundverschluss nach Anspruch 1, **dadurch gekennzeichnet**, das es sich bei den flüssigkeitsrückhaltenden und/oder flüssigkeitsabweisenden Mitteln um eine Schicht oder um eine Beschichtung handelt, und diese insbesondere in Form eines Randwulstes (45) ausgebildet ist.

3. Wundverschluss nach Anspruch 2, **dadurch gekennzeichnet, dass** die Schicht oder Beschichtung aus Kunststoff, insbesondere aus Polyurethan oder Polyethylen besteht.

4. Wundverschluss nach einem der vorhergehenden Ansprüche, **dadurch gekennzeichnet, dass** es sich bei den flüssigkeitsrückhaltenden und/oder flüssigkeitsabweisenden Mitteln um einen Schaumstoff handelt.

5. Wundverschluss nach einem der vorhergehenden Ansprüche, weiter **gekennzeichnet durch** eine zusätzliche, insbesondere wasserdampfdurchlässige Folie (37), die nach dem Verschließen der Wunde über dem Wundverschluss (31) anordenbar ist.

6. Wundverschluss nach einem der vorhergehenden Ansprüche, **dadurch gekennzeichnet, dass** das Halteelement (33) auf der den hakenartigen Elementen (35) abgewandten Seite Klebemittel (34) zur Anbringung des Halteelements am Wundrand, insbesondere eine Klebeschicht aufweist.

7. Wundverschluss nach einem der vorhergehenden Ansprüche, **dadurch gekennzeichnet, dass** das Halteelement eine Breite von weniger als 20 mm, insbesondere eine Breite zwischen 5 mm und 10 mm, und/oder einschließlich der hakenartigen Elemente eine Höhe zwischen 0,5 mm und 5 mm, insbesondere zwischen 0,5 mm und 1,5 mm, besitzt.

8. Wundverschluss nach einem der vorhergehenden Ansprüche, **dadurch gekennzeichnet, dass** die hakenartigen Elemente des Halteelements pilzkopfförmig ausgebildet sind und/oder die hakenartigen Elemente des Halteelements Widerhaken aufweisen, insbesondere harpunenförmig ausgebildet sind.

9. Wundverschluss nach einem der vorhergehenden Ansprüche, **dadurch gekennzeichnet, dass** es sich bei dem netzartigen Abdeckelement um ein Gewebe, insbesondere um sogenannten Tüll, handelt.

10. Wundverschluss nach einem der vorhergehenden Ansprüche, **dadurch gekennzeichnet, dass** das netzartige Abdeckelement ein Gewicht zwischen 10 und 100 g/m², vorzugsweise ein Gewicht zwischen 15 und 80 g/m², und/oder eine Reißkraftdehnung zwischen 30 % und 100 %, vorzugsweise zwischen 30 % und 60 %, besitzt.

11. Wundverschluss nach einem der vorhergehenden Ansprüche, **dadurch gekennzeichnet, dass** das netzartige Abdeckelement auf mindestens einer seiner beiden Seiten mindestens teilweise mit einer, insbesondere wasserdampfdurchlässigen Beschichtung oder Folie versehen ist.

12. Wundverschluss nach einem der vorhergehenden Ansprüche, **dadurch gekennzeichnet, dass** die Maschenweite des netzartigen Abdeckelements mindestens doppelt so groß ist wie der Durchmesser der hakenartigen Elemente des Halteelements, wobei vorzugsweise die Maschenweite des netzartigen Abdeckelements zwei bis vier Mal so groß ist wie der Durchmesser der hakenartigen Elemente des Halteelements.

13. Wundverschluss nach einem der vorhergehenden Ansprüche, **dadurch gekennzeichnet, dass** die Anordnung der hakenartigen Elemente auf dem Halteelement derart gewählt ist, dass zwischen 1 und 3 hakenartige Elemente in eine Masche des netzartigen Abdeckelements passen.

14. Wundverschluss nach Anspruch 13, **dadurch gekennzeichnet, dass**, bezogen auf die Anordnung des Halteelements am Wundrand, in Richtung des Wundverlaufs und in Richtung der auf die Wunde aufzubringenden Wundspannung des netzartigen Abdeckelements unterschiedlich viele hakenartige Elemente des Halteelements in eine Masche passen, und dabei insbesondere in horizontaler Richtung ein hakenartiges Element in eine Masche passt und in vertikaler Richtung zwei hakenartige Elemente in eine Masche passen.

15. Wundverschluss nach einem der vorhergehenden Ansprüche, **dadurch gekennzeichnet, dass** die Haltefestigkeit des Wundverschlusses an der Wunde bei Zugbelastung des netzartigen Abdeckelements senkrecht zum Wundverlauf mindestens 10 N, vorzugsweise zwischen 12 N und 20 N, beträgt.

## Claims

1. Adjustable wound closure device (11; 21; 31) for closing wounds, comprising
- at least one band-like or strip-like retaining element (12; 22; 33) provided for attachment in the region of at least one edge of a wound, wherein the retaining element (12; 22; 33) has a multiplicity of hook-like elements (13; 23; 35) on at least part of at least one of its two faces, and
- at least one gauze-like cover element (14; 24; 36), which is intended to substantially completely cover the wound and the edge regions provided with the retaining element,
- wherein the hook-like elements (13; 23; 35) of the retaining element (12; 22; 33) and the meshes (15; 25) of the cover element (14; 24; 36) are designed and arranged in such a way that the wound can be reversibly closed by a positive engagement of at least some of the hook-like elements in the meshes, and
- wherein the retaining element (42), on at least one of its two longitudinally extending edges, has liquid-retaining and/or liquid-repelling means (45) that can reduce or prevent movement of wound liquid, blood or the like out of the wound into the hook structure of the retaining element.

2. Wound closure device according to claim 1, **characterized in that** the liquid-retaining and/or liquid-repelling means are a layer or a coating, which is preferably designed in the form of an edge bead (45).

3. Wound closure device according to claim 2, **characterized in that** the layer or coating is composed of synthetic material, preferably made of polyurethane or polyethylene.

4. Wound closure device according to any of the preceding claims, **characterized in that** the liquid-retaining and/or liquid-repelling means are a foam.

5. Wound closure device according to any of the preceding claims, further **characterized by** an additional film (37), preferably a film permeable to water vapor, which can be arranged over the wound closure device (31) after the wound has been closed.

6. Wound closure device according to any of the preceding claims, **characterized in that** the retaining element (33), on the face directed away from the hook-like elements (35), has adhesive (34) for applying the retaining element to the wound edge, in particular an adhesive layer.

7. Wound closure device according to any of the preceding claims, **characterized in that** the retaining element has a width of less than 20 mm, in particular a width of between 5 mm and 10 mm, and/or including the hook-like elements, has a height of between 0.5 mm and 5 mm, in particular of between 0.5 mm and 1.5 mm.

8. Wound closure device according to any of the preceding claims, **characterized in that** the hook-like elements of the retaining element are shaped like a mushroom head, and/or the hook-like elements of the retaining element have barbs, in particular are harpoon-shaped.

9. Wound closure device according to any of the preceding claims, **characterized in that** the gauze-like cover element is a woven fabric, in particular a fabric called tulle.

10. Wound closure device according to any of the preceding claims, **characterized in that** the gauze-like cover element has a weight of between 10 and 100 g/m², preferably a weight of between 15 and 80 g/m², and/or an elongation at break of between 30% and 100%, preferably of between 30% and 60%.

11. Wound closure device according to any of the preceding claims, **characterized in that** the gauze-like cover element, on at least one of its two faces, is provided at least partially with a coating or film that in particular is permeable to water vapor.

12. Wound closure device according to any of the preceding claims, **characterized in that** the mesh width of the gauze-like cover element is at least twice as great as the diameter of the hook-like elements of the retaining element, wherein preferably the mesh width of the gauze-like cover element is two to four times as great as the diameter of the hook-like elements of the retaining element.

13. Wound closure device according to any of the preceding claims, **characterized in that** the arrangement of the hook-like elements on the retaining element is chosen in such a way that between 1 and 3 hook-like elements fit into one mesh of the gauze-like cover element.

14. Wound closure device according to claim 13, **characterized in that**, relative to the arrangement of the retaining element on the wound edge, different numbers of hook-like elements of the retaining element fit into one mesh in the direction of the wound profile and in the direction of the wound tensioning to be applied to the wound of the gauze-like cover element, preferably with one hook-like element fitting into one mesh in the horizontal direction and two hook-like elements fitting into one mesh in the vertical direction.

15. Wound closure device according to any of the preceding claims, **characterized in that** the holding strength of the wound closure device on the wound, upon tensile loading of the gauze-like cover element perpendicular to the wound profile, is at least 10 N, preferably between 12 N and 20 N.

## Revendications

1. Fermeture de plaie réglable (11 ; 21 ; 31) pour refermer des plaies, comprenant
- au moins un élément de fixation en forme de bande ou de ruban (12 ; 22 ; 33) qui est prévu pour être appliqué dans la région d'au moins un bord d'une plaie, l'élément de fixation (12 ; 22 ;33) présentant, sur au moins une partie d'au moins l'un de ses deux côtés, une pluralité d'éléments de type crochets (13 ; 23 ; 35) et
- au moins un élément de recouvrement de type filet (14 ; 24 ; 36), qui est prévu pour recouvrir essentiellement complètement la plaie et les régions de bord pourvues de l'élément de fixation,
- les éléments de type crochets (13 ; 23 ; 35) de l'élément de fixation (12 ; 22 ; 33) et les mailles (15 ; 25) de l'élément de recouvrement (14 ; 24 ; 36) étant réalisés et disposés de telle sorte que la plaie puisse être refermée de manière réversible par un engagement géométrique d'au moins une partie des éléments de type crochets dans les mailles, et
- l'élément de fixation (42) présentant, au niveau d'au moins l'un de ses deux bords s'étendant dans la direction longitudinale, des moyens (45) retenant les liquides et/ou repoussant les liquides, à l'aide desquels une pénétration de liquides de plaie, de sang ou similaire, hors de la plaie dans la structure de crochets de l'élément de fixation peut être réduite ou évitée.

2. Fermeture de plaie selon la revendication 1, **caractérisée en ce que** les moyens retenant les liquides et/ou repoussant les liquides sont constitués par une couche ou un revêtement réalisé(e) notamment sous forme de bourrelet de bord (45).

3. Fermeture de plaie selon la revendication 2, **caractérisée en ce que** la couche ou le revêtement se compose de plastique, en particulier de polyuréthanne ou de polyéthylène.

4. Fermeture de plaie selon l'une quelconque des revendications précédentes, **caractérisée en ce que** les moyens retenant les liquides et/ou repoussant les liquides sont constitués par une mousse.

5. Fermeture de plaie selon l'une quelconque des revendications précédentes, **caractérisée en outre par** une feuille supplémentaire (37), en particulier perméable à la vapeur d'eau, qui peut être disposée après la fermeture de la plaie pardessus la fermeture de plaie (31).

6. Fermeture de plaie selon l'une quelconque des revendications précédentes, **caractérisée en ce que** l'élément de fixation (33) présente, sur le côté opposé aux éléments de type crochets (35), des moyens adhésifs (34) pour appliquer l'élément de fixation sur le bord de la plaie, en particulier une couche d'adhésif.

7. Fermeture de plaie selon l'une quelconque des revendications précédentes, **caractérisée en ce que** l'élément de fixation présente une largeur inférieure à 20 mm, en particulier une largeur comprise entre 5 mm et 10 mm, et/ou, y compris les éléments de type crochets, une hauteur comprise entre 0,5 mm et 5 mm, en particulier comprise entre 0,5 mm et 1,5 mm.

8. Fermeture de plaie selon l'une quelconque des revendications précédentes, **caractérisée en ce que** les éléments de type crochets de l'élément de fixation sont réalisés en forme de tête de champignon, et/ou les éléments de type crochets de l'élément de fixation présentent des barbes, en particulier sont réalisés en forme de harpons.

9. Fermeture de plaie selon l'une quelconque des revendications précédentes, **caractérisée en ce que** l'élément de recouvrement de type filet est un tissu, en particulier ce qu'on appelle du tulle.

10. Fermeture de plaie selon l'une quelconque des revendications précédentes, **caractérisée en ce que** l'élément de recouvrement de type filet possède un poids compris entre 10 et 100 g/m², de préférence un poids compris entre 15 et 80 g/m², et/ou un étirement à la rupture compris entre 30 % et 100 %, de préférence compris entre 30 % et 60 %.

11. Fermeture de plaie selon l'une quelconque des revendications précédentes, **caractérisée en ce que** l'élément de recouvrement de type filet est pourvu sur au moins l'un de ses deux côtés, au moins en partie d'un revêtement ou d'un film, notamment perméable à la vapeur d'eau.

12. Fermeture de plaie selon l'une quelconque des revendications précédentes, **caractérisée en ce que** la largeur de maille de l'élément de recouvrement de type filet est au moins deux fois plus grande que le diamètre des éléments de type crochets de l'élément de fixation, la largeur de maille de l'élément de recouvrement de type filet étant de préférence deux à quatre fois plus grande que le diamètre des éléments de type crochets de l'élément de fixation.

13. Fermeture de plaie selon l'une quelconque des revendications précédentes, **caractérisée en ce que** l'agencement des éléments de type crochets sur l'élément de fixation est choisi de telle sorte qu'entre 1 et 3 éléments de type crochets s'ajustent dans une maille de l'élément de recouvrement de type filet.

14. Fermeture de plaie selon la revendication 13, **caractérisée en ce que**, par rapport à l'agencement de l'élément de fixation sur le bord de la plaie, dans la direction de l'étendue de la plaie et dans la direction de la couverture de plaie de l'élément de recouvrement de type filet devant être appliquée sur la plaie, un nombre différent d'éléments de type crochets de l'élément de fixation s'ajustent dans une maille et en l'occurrence en particulier, dans la direction horizontale, un élément de type crochet s'ajuste dans une maille et dans la direction verticale, deux éléments de type crochets s'ajustent dans une maille.

15. Fermeture de plaie selon l'une quelconque des revendications précédentes, **caractérisée en ce que** la tenue de la fermeture de plaie sur la plaie, en cas de sollicitations de traction de l'élément de recouvrement de type filet perpendiculairement à l'étendue de la plaie, est d'au moins 10 N, de préférence est comprise entre 12 N et 20 N.
